# EUROPEAN PATENT SPECIFICATION

(11) **EP 3 122 387 B1**
(45) Date of publication and mention of the grant of the patent: **06.06.2018**
(21) Application number: 15720438.9
(22) Date of filing: 26.03.2015
(51) Int. Cl.: A61L 2/26, B65G 35/00

(54) **DEVICE TO INTRODUCE AND EXTRACT CONTAINERS WITH RESPECT TO A STERILIZATION MACHINE AND STERILIZATION MACHINE COMPRISING SAID DEVICE**
VORRICHTUNG ZUR EINFÜHRUNG UND ENTNAHME VON BEHÄLTERN IN BEZUG AUF EINE STERILISATIONSMASCHINE UND STERILISATIONSMASCHINE MIT BESAGTER VORRICHTUNG
DISPOSITIF D'INTRODUCTION ET D'EXTRACTION DE RÉCIPIENTS DANS UNE MACHINE DE STÉRILISATION ET MACHINE DE STÉRILISATION COMPRENANT LEDIT DISPOSITIF

(30) Priority: 26.03.2014 IT UD20140054
(43) Date of publication of application: 01.02.2017
(73) Proprietor: Icos Pharma S.p.A., 33080 Zoppola (IT)
(72) Inventor: ZARDINI, Fabio, I-31033 Castelfranco Veneto (IT)
(74) Representative: Petraz, Gilberto Luigi
(86) International application number: PCT/IB2015/052229
(87) International publication number: WO 2015/145383

(56) References cited:
- EP-A1- 1 787 662
- EP-A2- 1 787 731
- WO-A1-2009/030599
- WO-A1-2012/076944
- JP-A- H05 294 441
- US-A1- 2007 205 081

## Description

### FIELD OF THE INVENTION

Forms of embodiment described here concern a device to introduce and extract containers, for example baskets containing objects to be sterilized or that have been sterilized, with respect to a sterilization machine.

Forms of embodiment described here also concern a sterilization machine, for example an autoclave, in which the objects housed in said containers, or containing baskets, are subjected to a sterilization treatment. In particular, they concern a sterilization machine in which the containers are fed and discharged automatically by said device.

Forms of embodiment described here can be used in the field of sterilization, for example, of medical instruments for hospital wards, operating theaters, laboratories, or other instruments for the pharmaceutical industry, or again, in general, objects whose use and storage require them to be sterile.

### BACKGROUND OF THE INVENTION

It is known that hospital structures are usually provided with a washing plant to carry out a treatment of pre-washing, washing, thermal-disinfection and sterilization of objects, such as for example instruments used in the operating theaters and therefore potentially infected and not sterile, before they can be reused.

In particular, known washing plants are normally divided into several sectors, isolated from each other for reasons of hygiene and respectively called "soiled", or reception sector, "clean" sector and "sterile" sector.

In the first sector, that is, the soiled one, baskets containing soiled objects to undergo various treatments arrive. In general the treatments carried out are pre-washing with cold water only, possible washing in an ultrasound bath, washing with hot water and possibly detergents, the necessary rinses, thermal disinfection and final drying.

Usually the pre-washing is carried out by a battery of suitable machines or pre-washing units, two or three for example, operating one after the other in series.

The cycle that provides washing, rinsing, thermal disinfection and drying, on the other hand, is carried out in a battery of suitable washing and thermal disinfection machines, for example five or six machines, operating in parallel with respect to each other.

Generally, each washing and thermal disinfection machine consists of a washing chamber with an aperture facing toward the soiled sector and an opposite aperture facing toward the clean sector.

The baskets with the objects to be washed are removed at the exit of the last pre-washing unit and fed to the washing and thermal disinfection machine available at that moment.

After having been thermally disinfected and dried, the objects pass into the second sector, that is, the clean sector, in which they are possibly put into envelopes and, from here, are fed to a sterilization apparatus, in which a battery of sterilization machines, generally autoclaves, sterilize the objects.

Normally, the sterilization apparatus includes a plurality of sterilization machines that operate in parallel and that are typically disposed adjacent to each other to define a substantially aligned disposition of the respective entrance or loading apertures of the baskets, and/or the respective exit or discharge apertures of the baskets.

Sterilization machines are conventionally the "pass-through" or tunnel type, that is, they include a loading aperture and a discharge aperture positioned on opposite sides of the internal treatment chamber and aligned in a direction of feed and discharge that will be identified hereafter for reasons of convenience as "direction of feed".

This allows to supply greater continuity to the treatment process of the objects, and, at the same time, to keep separate the entrance and exit zone of the baskets containing objects, thus achieving the desired separation of the clean sector from the sterile sector.

It is known that on both the loading side and discharge side, sterilization machines are provided with a watertight closing door that allows to separate the inside of the treatment chamber from the outside during sterilization.

This is due to the fact that, for a correct sterilization, the contamination of the treatment chamber must be prevented, the leakage of treatment fluids must be avoided and the desired internal conditions must be maintained during sterilization. Moreover, the watertight closing doors allow to prevent the leakage of material due to possible internal super pressure, and seal the inside of the treatment chamber also for safety reasons, in consideration of the fact that during sterilization high treatment temperatures can be reached, for example comprised between 120°C and 135°C.

The feed and discharge of the baskets, both entering the sterilization machines and exiting from them, is obtained by movement devices that determine the translation of the baskets in the direction of feed.

To this purpose, the movement devices can be provided with a positioning device, such as a translator slider, mobile in the direction of feed to both feed the baskets inside the treatment chambers of sterilization machines, and also to discharge them at the end of sterilization.

Moreover, the movement devices can be provided with motorized guide members that allow the linear translation of the baskets.

It is also known that in washing plants comprising a battery of sterilization machines, movement devices are present, mobile in a direction of movement substantially orthogonal with respect to said direction of feed in order to position itself on each occasion in correspondence to the respective loading or discharge aperture, depending on the specific function carried out.

Known sterilization machines can have guide means inside the treatment chambers, configured to guide the baskets inside the treatment chambers during feed and discharge.

The guide means are passive since, for obvious reasons linked to the nature of sterilization treatments, the presence of motorized elements inside the treatment chamber, is precluded.

Both in proximity to the loading aperture and in proximity to the discharge aperture, each sterilization machine normally has an interspace between the treatment chamber and the movement device, in particular the corresponding external guide means.

A respective closing door is inserted in the interspace, configured to seal the corresponding aperture.

It can be provided that the closing doors remain inside the respective interspaces during their movement and positioning toward a closed position and from the latter toward a disengaged position of the corresponding aperture. This movement generally provides that the closing doors slide on a lying plane orthogonal to the direction of feed.

Generally, the size of the interspaces, measured in the direction of feed, can even be bigger than 100 mm, for example in the order of 150 mm or more.

The interspace between external guide means and treatment chamber can obstruct, and even compromise, the movement of the baskets containing the objects to be sterilized due to the lack of support and guide to which they are subjected during their movement travel.

Indeed the baskets can divert from their lying plane in correspondence with the interspaces due to the effect of their own weight and/or the weight of the objects inside them, and tend to incline toward the inside of the interspaces.

This can determine a blockage of the baskets during their movement if the inclination is sufficient to determine contact of the baskets against the front wall of the sterilization machine, during feed, or against the movement device, during the discharge of the baskets.

The probability of blockages increases with the reduction in the ratio between size of the baskets and that of the interspace, or with the increase in weight of the objects in the baskets.

A possible blockage of the baskets can cause damage to the structures of the washing plant, as well as determining stoppages of the machine and extended times for the washing cycles that can also be due to the need to repeat some steps of the process.

One disadvantage of known movement devices is that they are not able to prevent, efficiently, safely and automatically, the onset of blockages caused by the interspace between the external guide means and the treatment chamber.

Documents WO-A-2009/030599, WO-A-2012/076944, EP-A-1.787.731 and EP-A-1.787.622 are known, which describe batteries of aligned washing machines, served frontally by a slider translatable parallel to the direction of alignment of the washing machines themselves.

Document US-A-2007/0205081 describes a rotating board of a transport system for a unit producing semiconductors.

Document JP-A-05294441 describes a cart able to serve an operating section selectively closed by a door; the cart is provided with a conveyor to deliver or remove a load with respect to the operating zone.

There is therefore a need to perfect a device to introduce and extract containers with respect to a sterilization machine that can overcome at least one of the disadvantages of the state of the art.

In particular, one purpose of the present invention is to make a movement device, to introduce and extract containers, or baskets, containing objects to be sterilized, or that have been sterilized, with respect to a sterilization machine, that is able to feed and/or discharge said baskets automatically, quickly and efficiently, toward and from one or more sterilization machines without the risk of the baskets getting blocked during said movement.

Another purpose of the present invention is to make a sterilization machine for objects with a movement device of the baskets containing said objects that is simple to achieve and is able to move the baskets into and/or out of the treatment chamber automatically, efficiently and effectively, without any risk of the baskets getting blocked.

It is also a purpose of the present invention to make a sterilization apparatus that allows, automatically, reliably and without the risk of blockages, both to feed the baskets containing objects to be treated in a battery of sterilization machines and also to discharge said baskets.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

Forms of embodiment described here concern a device which can be used to introduce and extract containers of objects, to be sterilized or that have been sterilized, with respect to a sterilization machine in a direction of feed. The device comprises a container-loading plane defining a movement path, and motorized linear guide rollers supported by a support frame, disposed along the container-loading plane and configured to move the containers in a guided manner along the movement path in the direction of feed.

According to one form of embodiment, the device also comprises:
- an auxiliary guide extension connected to the container-loading plane comprising a frame connected to the support frame, at least one abutment element and auxiliary guide rollers. The frame is slidingly mobile between a first retracted position, in which the frame is comprised in the bulk of the container-loading plane, aligned or more internal with respect to a front edge of the support frame and in which the auxiliary guide rollers are coplanar with respect to the motorized linear guide rollers, and a second operating position, in which the frame is at least partly protruding from the container-loading plane in order to define an extension of the movement path;
- a translator slider mobile in the direction of feed to selectively extract or insert the containers with respect to the machine, and provided with a thrust element configured to interfere with the bulk of the abutment element and configured to contact the abutment element and automatically thrust the auxiliary guide extension toward the outside of the container-loading plane in the operating position due to the effect of the contact.

In this way, the advantage is obtained of being able to use, when necessary, a movement path of containers which is extended with respect to that defined by the only one loading plane in which there are motorized linear guide rollers. This allows to support the containers with greater efficiency where there is, for example, an interspace to be passed over between the loading plane and the destination of the containers defined by the sterilization machine.

According to forms of embodiment described here, the device can comprise automatic thrust means and automatic return means of the auxiliary guide extension mobile in the direction of feed to move the auxiliary guide extension between the retracted position and the operating position, and vice versa.

The advantage is thus obtained, by automatically moving the auxiliary guide extension, of reducing the cycle times of the sterilization process, as well as automatically ensuring an extended support to the containers during their movement, increasing the efficiency and safety of this movement.

Other forms of embodiment described here concern a machine for sterilizing objects contained in containers, comprising:
- a treatment chamber provided with at least one container-passage aperture in a direction of feed,
- a closing door mobile on a lying plane transverse to this direction of feed to close the container-passage aperture,
- a device to introduce and extract the containers with respect to the treatment chamber in the direction of feed according to the present description.

Forms of embodiment described here also concern an apparatus for sterilizing objects contained in containers that comprises a battery of machines for sterilizing the objects, according to the present description.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the description or in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some forms of embodiment, given as a non-restrictive example with reference to the attached drawings wherein:
- figs. 1 to 4 are partly sectioned schematic views of a sterilization machine provided with a movement device according to the present invention;
- fig. 5 is an enlarged view of detail A in fig. 2;
- fig. 6 is an enlarged view of detail B in fig. 3;
- fig. 7 is an enlarged view of detail C in fig. 3;
- fig. 8 is an enlarged view of detail D in fig. 4;
- fig. 9 is a lateral view of a detail of the device in fig. 1;
- fig. 10 is a schematic plan view of a washing plant according to the present invention;
- fig. 11 is a variant of fig. 10.

In the following description, the same reference numbers indicate identical parts of the sterilization machine according to the present invention, also in different forms of embodiment. It is understood that elements and characteristics of one form of embodiment can be conveniently incorporated into other forms of embodiment without further clarifications.

### DETAILED DESCRIPTION OF SOME FORMS OF EMBODIMENT

We shall now refer in detail to the various forms of embodiment of the present invention, of which one or more examples are shown in the attached drawing. Each example is supplied by way of illustration of the invention and shall not be understood as a limitation thereof. For example, the characteristics shown or described insomuch as they are part of one form of embodiment can be adopted on, or in association with, other forms of embodiment to produce another form of embodiment. It is understood that the present invention shall include all such modifications and variants.

Before describing these forms of embodiment, we must also clarify that the present description is not limited in its application to details of the construction and disposition of the components as described in the following description using the attached drawings. The present description can provide other forms of embodiment and can be obtained or executed in various other ways. We must also clarify that the phraseology and terminology used here is for the purposes of description only, and cannot be considered as limitative.

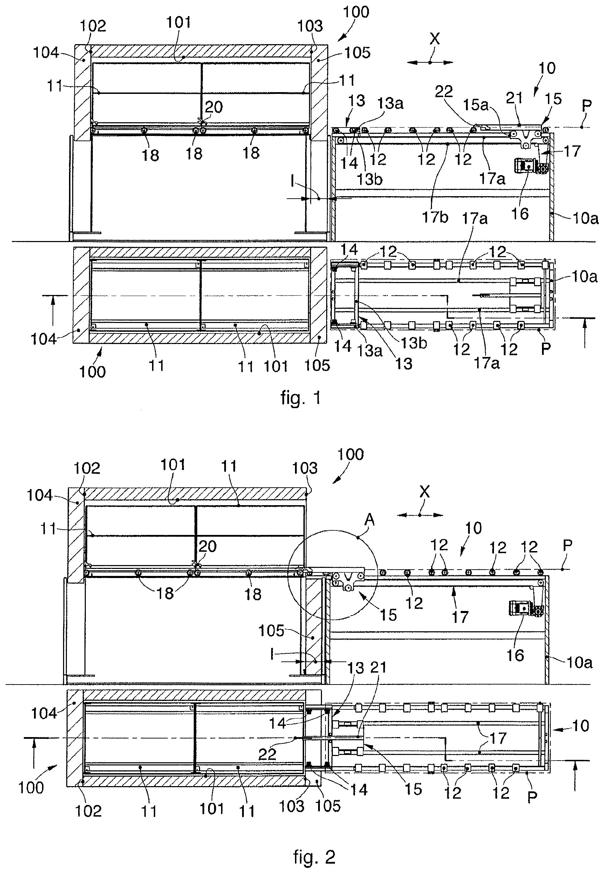

Figs. 1 to 4 are used to describe forms of embodiment of a movement device 10, in this case associated with a sterilization machine, such as an autoclave 100.

Figs. 1 to 4 each show a front view and a plan view of the autoclave 100 and the device 10, partly sectioned and deliberately simplified so as to clarify the description.

The autoclave 100 is configured to perform the sterilization of objects, for example medical instruments for hospital wards, operating theaters, laboratories or other instruments for the pharmaceutical industry, or again, in general, objects whose use and/or storage requires them to be sterile, housed in containers or baskets 11.

The device 10 is configured to introduce and extract the baskets 11 with respect to the autoclave 100 in a direction of feed X and according to a substantially linear movement path defined by a container-loading plane P.

The device 10 includes motorized linear guide members, for example motorized rollers 12 which can be driven in a known way by a kinematic chain comprising an electric motor and a transmission member, for example with chain or belt, not shown in the drawings.

The device 10 can also include a support frame 10a to which are connected and by which are supported the functional components of the device 10, such as the motorized rollers 12, the components of the kinematic chain, and other components that will be mentioned and described hereafter.

The motorized rollers 12 can be disposed along the container-loading plane P and be aligned on two or more rows parallel to the direction of feed X to define the movement path. When the baskets 11 are positioned in contact with the motorized rollers 12, these act as motorized guide members for the baskets 11 toward and away from the autoclave 100, according to a guided movement.

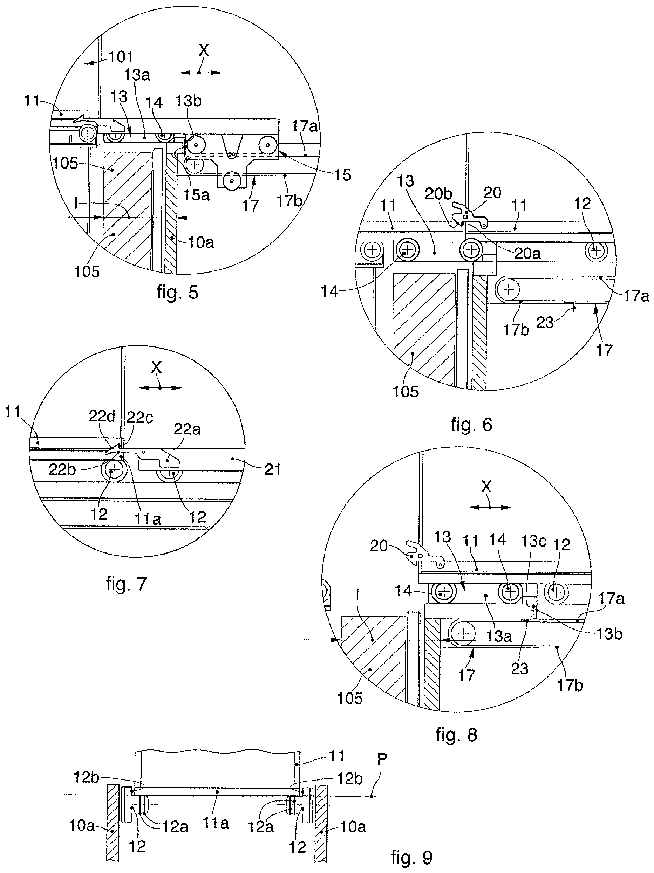

Fig. 9 is used to describe possible forms of embodiment of the motorized rollers 12, in which they are supported by the support frame 10a and have drawing elements, for example silicone rings 12a, configured to contact a base 11a of the baskets 11 and to draw them by friction.

In fact, the rotation of the motorized rollers 12 allows the translation of the baskets 11 by the effect of contact of the base 11a with the silicone rings 12a.

Moreover, the support plane of the base 11a defined by the motorized rollers 12 coincides with the container-loading plane P.

The shape of the motorized rollers 12, moreover, provides lateral shoulders 12b configured to guide the baskets 11 and prevent them being positioned transverse to the direction of feed X.

The autoclave 100 includes a treatment chamber 101 inside which the sterilization operations are performed on the objects contained in the baskets 11.

Inside the treatment chamber 101 one or more baskets 11 can be housed, possibly connected to each other according to size.

Figs. 1 to 4 are used to describe forms of embodiment in which the autoclave 100 is the "pass-through" type, in which the treatment chamber 101 is provided with a first container-passage aperture, or loading aperture 102, and a second container-passage aperture, or discharge aperture 103, aligned in the direction of feed X on opposite sides of the treatment chamber 101.

In these forms of embodiment, it is provided that the baskets 11 are fed in the treatment chamber 101 through the loading aperture 102 before sterilization, and discharged from the treatment chamber 101 through the discharge aperture 103 at the end of sterilization.

This solution is described here merely by way of example and is not intended to limit the present invention, since it is easily adaptable by a person of skill in the art for other solutions as well, for example in which the autoclave 100 is the "single door" type, that is, it has a single container-passage aperture both for feeding and for discharging the baskets 11.

Figs. 1 to 4 show a device 10 positioned in proximity to the discharge aperture 103 of the autoclave 100 and configured to extract the baskets 11 from it.

What is described hereafter referring to this type of device 10 is also referred in the same way to devices configured to introduce the baskets into the autoclave 100 through the loading aperture 102.

The autoclave 100 comprises two closing doors, one for each of the loading 102 and discharge 103 apertures, and respectively indicated with the reference numbers 104 and 105.

The closing doors 104 and 105 are mobile along their own lying plane transverse to the direction of feed X.

In some forms of embodiment, it can be provided that the closing doors 104 and 105 are translatable along a plane orthogonal to the direction of feed X, for example vertically or horizontally.

In particular, the closing doors 104 and 105 have the function of hermetically closing the respective loading 102 and discharge 103 apertures, during the sterilization of the objects contained in the baskets 11.

Once sterilization is terminated, the closing doors 104 and 105 free the respective passage apertures 102 and 103, to allow both the insertion and extraction of the baskets 11 to/from the treatment chamber 101.

Fig. 1 is used to describe a possible example positioning of the closing doors 104 and 105 in a closed position, in which the hermetic closing of the loading 102 and discharge 103 apertures is guaranteed.

Figs. 2 to 4 show by way of example a positioning of the closing door 105 in an open position, in which the closing door 105 is lowered with respect to the discharge aperture 103.

With reference to figs. 1 to 4, the device 10 is distanced from the discharge aperture 103, in the direction of feed X, in order to define a passage interspace I for the closing door 105.

The interspace I is intended for the passage of the closing door 105 from the open position to the closed position and can house the closing door 105, completely or partly, when it is in the open or closed position.

Figs. 1 to 4 are used to describe forms of embodiment in which the device 10 includes an auxiliary guide extension 13, connected to the container-loading plane P and mobile with respect to it and to the motorized rollers 12.

The auxiliary guide extension 13 can comprise a frame 13a connected to the support frame 10a, in particular to the container-loading plane P, and can comprise one or more auxiliary guide rollers 14, in this case two.

In possible solutions, the frame 13a is sliding with respect to the container-loading plane P in the direction of feed X from a first retracted position, shown by way of example in fig. 1, to a second operating position, shown by way of example in figs. 2 to 4.

In the retracted position, the auxiliary guide extension 13, in particular the frame 13a, is comprised in the bulk of the container-loading plane P, that is, aligned or more internal with respect to a front edge of the support frame 10a.

The auxiliary guide extension 13 is intended comprised in the bulk of the container-loading plane P both when it is completely recessed inside said plane and also when it is at least mostly recessed inside said plane.

In the retracted position, the auxiliary guide extension 13 must be at least mostly external to the interspace I, and in any case must allow the free movement of the closing door 105 and the corresponding passage from the closed position to the open position and vice versa.

In fact, any interference between the auxiliary guide extension 13 and the movement of the closing door 105 could cause damage to both.

In the retracted position, the auxiliary guide extension 13 can have its own auxiliary guide rollers 14 coplanar to the motorized rollers 12.

In the operating position, obtained by translating the auxiliary guide extension 13 in the direction of feed X, the auxiliary guide extension 13 itself is at least partly protruding from the container-loading plane P, to define an extension of the movement path defined by the container-loading plane P. This extension is at least partly contained in the interspace I, so that the auxiliary guide extension 13 allows to at least partly close the interspace I in order to give a support and guide to the baskets 11, which is effective in contrasting the possibility that they could get blocked against the support frame 10a of the device 10 during their extraction from the treatment chamber 101.

In the operating position of the auxiliary guide extension 13, the auxiliary guide rollers 14 are located parallel but not coplanar to the motorized rollers 12 in the direction of feed X, that is, they are located on a plane which is parallel and different to the plane on which the motorized rollers 12 are located.

In possible forms of embodiment, not shown in the drawings, it can be provided that the frame 13a is connected to the support frame 10a and to the container-loading plane P in a rotatable manner around an axis of rotation orthogonal to the direction of feed X. In these forms of embodiment, in the retracted position, the auxiliary guide extension 13 can be located above or below the container-loading plane P.

The rotation of the auxiliary guide extension 13 around this axis of rotation can determine the passage thereof from the retracted position to the operating position and vice versa.

The device 10 includes a translator slider 15 mobile in a guided manner in the direction of feed X.

In some forms of embodiment, the device 10 can include an actuation element to move the translator slider 15. Usually, an actuation element, as used in association with forms of embodiment described here, can be an intrinsically linear movement actuator or be configured to convert a circular movement into a linear movement. The conversion can be commonly achieved by means of types of mechanism selected from a group consisting of: screw actuators, such as a jackscrew, actuators with ball screws and roll screws, or wheel and axle, for example drum, gear, pulley or shaft, actuators, like a lifting cable, a hoist, a rack and a pinion unit, a chain transmission, a belt transmission, actuators with rigid chain and a rigid belt. The actuation element can comprise a drive member configured to move the translator slider 15, which is made to function by a source of energy, for example an electric current, a hydraulic fluid pressure or pneumatic pressure. The drive member can be provided with a drive shaft, and configured to determine the desired movement of the translator slider 15. A drive member as used in association with the forms of embodiment described here can be a drive member chosen from a group comprising: an electric motor, a step electric motor, a magnetic motor, a linear axis with a motor, a linear motor, such as a mechanical linear motor, a piezo electric linear motor, an electro-magnetic linear motor, an electro-mechanical motor, an electro-magnet, a motor reducer, in particular a continuous current motor reducer. In possible implementations, the drive member can be a drive motor 16, for example an electric motor. The device 10 can also provide at least one motion transmission device, for example at least one motion transfer device 17. The motion transmission device can cooperate with the actuation element to move the translator slider 15. Examples of motion transfer device 17 are transmission members with chain or belt. For example the motion transmission device 17 can be connected to the drive member of the actuation element, for example to the drive motor 16 and also to the translator slider 15. The at least one transfer device 17 is driven by the drive motor 16 and transmits the motion imparted by the drive motor 16 to the translator slider 15. The at least one transfer device 17 can be the closed ring type and define a closed trajectory having an upper segment 17a rectilinear and parallel both to the container-loading plane P and to the direction of feed X. In some forms of embodiment, the at least one transfer device 17 also defines a lower segment 17b of trajectory which, connected to the upper segment 17a, completes the closed ring development of the latter.

In the specific case described with reference to figs. 1 to 4, the device 10 includes a pair of transfer devices 17, each of which can be configured as described above.

The upper segments 17a of the trajectories of the transfer devices 17 act as rectilinear guides along which the translator slider 15 slides in a guided manner in the direction of feed X.

In other forms of embodiment, a linear actuator can be provided, such as a jack, a piston, a rack or other, instead of the drive motor 16, provided with a mobile member connected to the translator slider 15 instead of the transfer devices 17.

Fixed rectilinear guides can also be provided, integrated with the support frame 10a and separate from the transfer devices 17 or from the linear actuators.

The translator slider 15 can be provided frontally with a thrust element, such as a thrust surface 15a, with a shape and position, below the container-loading plane P, coordinated with the shape and position of an abutment element, such as an abutment surface 13b, with which the frame 13a of the auxiliary guide extension 13 is provided at the rear.

The movement of the translator slider 15 toward the treatment chamber 101 in order to insert the baskets 11 therein, or in preparation for the extraction thereof, determines the contact between the thrust surface 15a and the abutment surface 13b, and the consequent thrust, due to the effect of this contact, of the auxiliary guide extension 13 toward the autoclave 100.

This thrust allows the automatic passage of the auxiliary guide extension 13 from the retracted position to the operating position.

The contact and the passage of the position described above are shown, by way of example, in figs. 2 and 5.

Once the translator slider 15 has been positioned in proximity to the discharge aperture 103, it is attached to the basket 11 to be extracted in a part of the latter located in correspondence to the same discharge aperture 103.

Then, the translator slider 15 is moved backwards in the direction of feed X to complete the extraction of the baskets 11.

At the end of this movement of the translator slider 15, the auxiliary guide extension 13 is automatically made to translate into the retracted position.

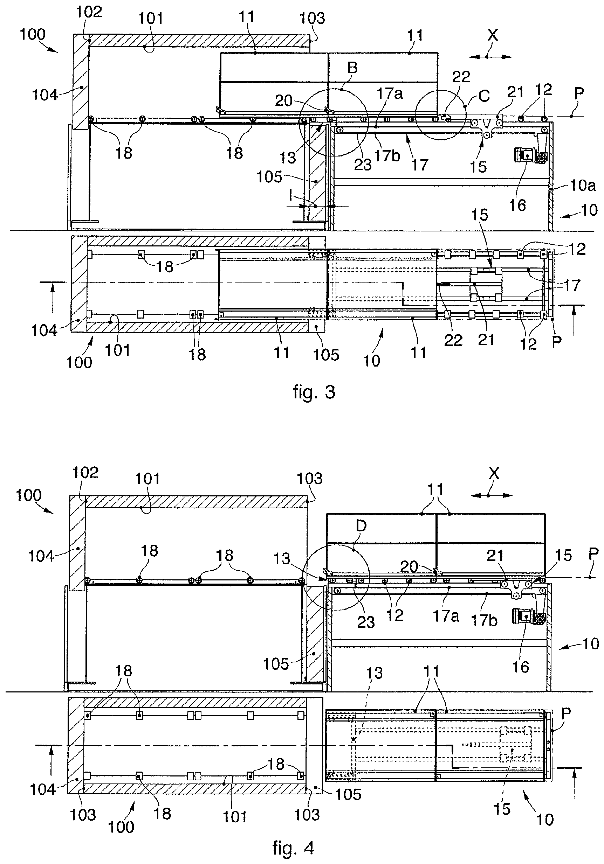

Figs. 3 and 7 are used to describe an intermediate translation step of the translator slider 15, while figs. 4 and 8 describe the final step of the translation, in which the auxiliary guide extension 13 is completely outside the interspace I.

To allow the movement of the baskets 11 inside the treatment chamber 101, the autoclave 100 includes, inside the treatment chamber, idle linear guide means, for example idle rollers 18, which define an internal guide path.

The idle rollers 18 are aligned with the motorized rollers 12 and connected to them by means of the auxiliary guide rollers 14, when the auxiliary guide extension 13 is in the operating position.

In this way, the overall path of the baskets 11 is defined by the succession of the movement path defined by the container-loading plane P, the extension defined by the auxiliary rollers 14 and the internal path.

A continuous guide of the baskets 11 is thus obtained, using only motorized members outside the treatment chamber 101, and, at the same time, the baskets 11 of reduced size are prevented from getting blocked against the support frame 10a.

With reference to figs. 3 and 6, whenever the loading of a plurality of baskets 11 is provided in the treatment chamber 101, these can be reciprocally connected by means of an attachment member 20, positioned at one end of each basket 11.

Fig. 6 shows, by way of example, an attachment member 20 with a first end pivoted to a first basket 11 and a second end, opposite the first end, provided with a hook 20a configured to contact and retain a second basket 11 during the translation of the first basket 11 drawn by the translator slider 15.

The attachment member 20 is also provided with a cam profile 20b protruding from the corresponding first basket 11 to enter into contact with the second basket 11 when this is brought near the first basket 11.

This contact determines the sliding of the cam profile 20b on the second basket 11 and the consequent rotation of the attachment member 20 with respect to its own pivoting to the first basket 11, lifting the hook 20a.

When the baskets 11 are brought even closer together, this determines the movement by gravity of the hook 20a, and the attachment of the baskets 11.

In some forms of embodiment, described for example with reference to figs. 1 to 4 and 7, the translator slider 15 includes a drawing arm 21 provided with a gripping member 22 at its terminal end.

When the translator slider 15 is positioned in correspondence to the discharge aperture 103, the drawing arm 21 is inserted at least partly inside the treatment chamber 101, below the base 11a of the basket 11 nearest the discharge aperture 103 itself, and the gripping member 22 is positioned in contact and in interference with the base 11a.

When the translator slider 15 is retracted, the drawing arm 21 draws the basket 11 toward the outside of the treatment chamber 101 due to the effect of this interference between the gripping member 22 and the basket 11.

In possible implementations, the drawing of one basket 11 can determine the drawing of all the baskets 11 present in the treatment chamber 101 and reciprocally connected by means of respective attachment members 20.

Fig. 7 is used to describe possible forms of embodiment of the gripping member 22, which can be pivoted to the drawing arm 21 and provided with a first portion 22a and a second portion 22b positioned on opposite sides of the pivoting point in the direction of feed X.

The first portion 22a and the second portion 22b can have different weights, for example the second portion 22b can be lighter, or they can have a different mass distribution with respect to the pivoting point, to define an asymmetrical balance condition of the two portions 22a and 22b with respect to the pivoting.

For example, a normally raised position of the second portion 22b with respect to the first portion 22a can be provided.

The second portion 22b can include a gripping tooth 22c, protruding from the upper part of the second portion 22b and transverse with respect to the container-loading plane P and to the direction of feed X, defining an angle close to a right angle with respect to them.

The second portion 22b can also include a cam profile 22d, configured to slide on the base 11a of the basket 11 during the motion of the translator slider 15 toward the treatment chamber 101 and the corresponding motion of the drawing arm 21 inside it. The sliding of the cam profile 22d on the base 11a of the basket 11 determines the rotation of the gripping member 22 with respect to its own pivoting point, and a lowering of the second portion 22b with corresponding lifting of the first portion 22a.

Since the gripping member 22 is configured to have a balanced position in which the first portion 22a is lowered, once the cam profile 22d is disengaged from the base 11a of the basket, the gripping member 22 automatically returns to its own balanced position, allowing the gripping tooth 22c to position itself in interference with the plane of the base 11a itself.

In this way, retracting the translator slider 15, and therefore the drawing arm 21 to which the gripping tooth 22c is connected, the drawing toward the outside of the treatment chamber 101 of the basket 11 is obtained due to the effect of the contact between the gripping tooth 22c and the base 11a.

In some solutions, it can be provided that the baskets 11 are extracted using a movement of the translator slider 15 of the "pilgrim step" type, that is, alternating its advance toward the treatment chamber 101 with its distancing from it.

Each advance is intended to bring the gripping tooth 22c to grip on a subsequent basket 11, while each distancing, after an advance, allows to extract by a desired quantity one or more baskets 11 from the treatment chamber 101.

Figs. 6 and 8 are also used to describe example forms of embodiment of the device 10 in which it also includes a return element 23, attached to the transfer device or devices 17, and solidly mobile with it along the corresponding ring trajectory.

In particular, the return element 23 protrudes from the transfer device 17 substantially orthogonally to the trajectory by a quantity sufficient to contact, when positioned in the upper segment 17a, a support wall 13c of the frame 13a.

The support wall 13c is located in the lower part of the frame 13a of the auxiliary guide extension 13 and acts as an abutment element between the return element 23 and the auxiliary guide extension 13 itself.

The contact between the return element 23 and the support wall 13c allows to automatically move the auxiliary guide extension 13 from its operating position to the retracted position by moving the translator slider 15 away from the treatment chamber 101.

In this way, during the discharge of the baskets 11 from the autoclave 100, the removal of the auxiliary guide extension 13 from the interspace I is automatically obtained as well, thus allowing the immediate movement of the closing door 105.

This is functional not only to extracting the baskets 11, preventing them from getting blocked on the support frame 10a of the device 10, but also to speeding up the treatment process, reducing the downtimes between the discharge of the baskets 11 and hermetic closing of the discharge aperture 103 to allow a subsequent sterilization.

Moreover, on the basis of the above, the thrust surface 15a and the return element 23 respectively perform the function of thrust means and return means.

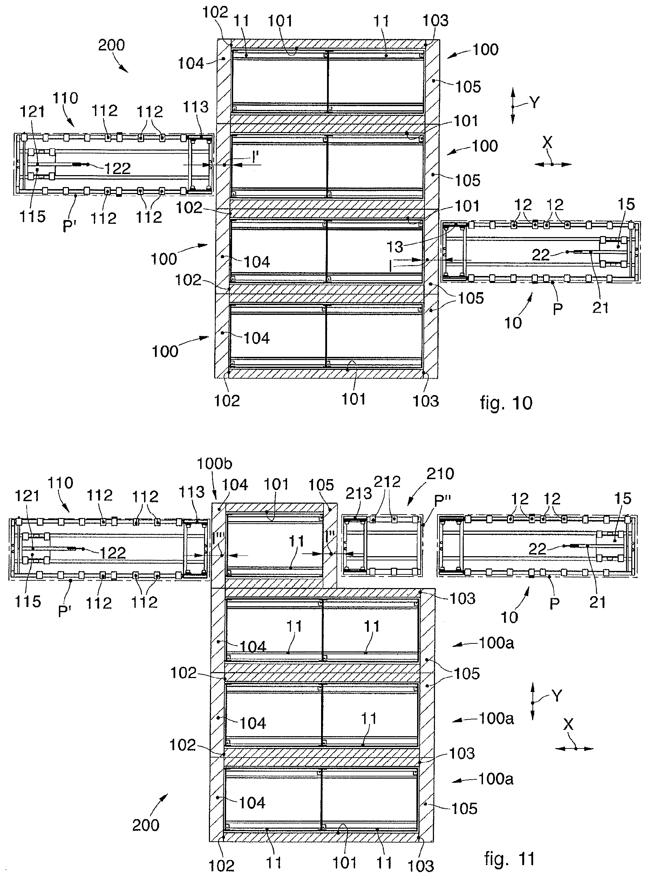

Fig. 10 is used to describe forms of embodiment of an apparatus 200 for sterilizing objects contained in baskets 11 which includes a battery of autoclaves 100 adjacent in parallel in a work direction Y, transverse, for example perpendicular, to the direction of feed X.

The autoclaves 100, of which there are four in the example shown, can be disposed so that the respective loading 102 and/or discharge 103 apertures are aligned in said work direction Y.

The apparatus 200 includes a device 10 for extracting baskets 11 that serves the discharge aperture 103 and slides in the work direction Y frontally with respect to the autoclaves 100, to position itself on each occasion, depending on the needs of the sterilization cycle, in correspondence to the discharge aperture 103 of an autoclave 100.

The device 10 includes, as described above, motorized rollers 12, an auxiliary guide extension 13 and a translator slider 15 mobile in the direction of feed X to extract the baskets 11 contained in the treatment chamber 101 of each autoclave 100, preventing them from getting blocked.

In some forms of embodiment, the apparatus 200 can also include a device 110 to introduce the baskets 11, completely similar to the device 10 and positioned on the loading side of the autoclaves 100, that is, on the opposite side with respect to the device 10.

The device 110, serving the loading aperture 102, is able to slide in the work direction Y according to a trajectory parallel to that of the device 10, to position itself on each occasion in correspondence to the loading aperture 102 of an autoclave 100.

The device 110, in a similar way to the device 10, is provided with motorized rollers 112, disposed on container-loading plane P', an auxiliary guide extension 113 mobile with respect to the container-loading plane P', and a translator slider 115 translatable in the direction of feed X to insert the baskets 11 into the treatment chamber 101 of each autoclave 100. The auxiliary guide extension 113 is made essentially like the auxiliary guide extension 13 described using figs. 1-9 for example.

In some forms of embodiment, the translator slider 115 can include a thrust arm 121 provided with a gripping member 122 that differs from the gripping member 22 of the drawing arm 21 of the device 10 in that it is configured to thrust the baskets 11, instead of drawing them.

The auxiliary guide extension 13 is configured to overlap the interspace I between the container-loading plane P and the discharge aperture 103, while the auxiliary guide extension 113 is configured to overlap an interspace I' comprised between the container-loading plane P' and the loading aperture 102.

In variant forms, described by way of example with reference to fig. 11, the apparatus 200 can include a battery of first autoclaves 100a, all equal to each other and having loading 102 and discharge apertures 103 aligned in the work direction Y, and one or more second autoclaves 100b, smaller in size than the first autoclaves 100a.

This configuration can be used, for example, when the sterilization process provides loads of objects, and therefore baskets 11, smaller than the capacity of the first autoclaves 100a, or a partial feed of baskets 11 to the apparatus 200, or a reduced frequency of said feed, as can happen, for example, during the night shift of a hospital structure.

The apparatus 200, in such variant forms, can provide that the loading aperture 102 of the second autoclave 100b is aligned to the loading aperture 102 of the first autoclaves 100a.

Alternatively, it can be provided to align the discharge aperture 103 of the second autoclave 100b and the discharge apertures 103 of the first autoclaves 100a.

With reference to fig. 11, an auxiliary device 210 can be provided, similar to the device 10 and the device 110, interposed between the latter and the second autoclave 110b and provided at least with its own motorized rollers 212 disposed along a container-loading plane P", and with an auxiliary guide extension 213 mobile with respect to said container-loading plane P". The auxiliary guide extension 213 is essentially made like the auxiliary guide extension 13 described using figs. 1-9 for example.

The auxiliary device 210 is configured to guide the baskets 11 from the treatment chamber 101 of the second autoclave 100b toward the device 110.

In particular, the auxiliary guide extensions 13 and 213 respectively allow to prevent the baskets 11 getting blocked in the passage from the device 210 to the device 10 and in the passage from the treatment chamber 101 to the device 210.

The auxiliary guide extension 13 is configured to overlap the interspace I between the container-loading plane P and the discharge aperture 103 of the first autoclaves 100a and between the container-loading planes P and P".

The auxiliary guide extension 113 is configured to overlap an interspace I' comprised between the container-loading plane P' and the loading aperture 102.

The auxiliary guide extension 213 is configured to overlap an interspace I" comprised between the container-loading plane P" and the discharge aperture 103 of the second autoclave 100b.

In these forms of embodiment, it can be provided that the drawing arm 21 of the device 10 is extendable, for example telescopic, in order to reach the baskets 11 inside the treatment chamber 101 of the second autoclave 100b.

It is clear that modifications and/or additions of parts may be made to the device 10, 110, 210 and to the autoclave 100, 100a, 100b as described heretofore, without departing from the field and scope of the present invention.

## Claims

1. Device to introduce and extract containers (11) with respect to a sterilization machine (100, 100a, 100b) in a direction of feed (X), comprising
- a container-loading plane (P, P', P") defining a movement path, and motorized linear guide rollers (12, 112, 212), supported by a support frame (10a), disposed along the container-loading plane (P, P', P") and configured to move said containers (11) in a guided manner along said movement path in the direction of feed (X),
**characterized in that** it also comprises
- an auxiliary guide extension (13, 113, 213) connected to said container-loading plane (P, P', P") and comprising a frame (13a) connected to the support frame (10a), at least one abutment element (13b) and auxiliary guide rollers (14), said frame (13a) being slidingly mobile between a first retracted position, in which said frame (13a) is comprised in the bulk of the container-loading plane (P, P', P"), aligned or more internal with respect to a front edge of the support frame (10a) and in which the auxiliary guide rollers (14) are coplanar with respect to the motorized linear guide rollers (12, 112, 212), and a second operating position, in which said frame (13a) is at least partly protruding from the container-loading plane (P, P', P") in order to define an extension of said movement path;
- a translator slider (15, 115) mobile in said direction of feed (X) to selectively extract or insert said containers (11) with respect to said machine (100, 100a, 100b), and provided with a thrust element (15a) configured to interfere with the bulk of said abutment element (13b) and configured to contact said abutment element (13b) and automatically thrust said auxiliary guide extension (13, 113, 213) toward the outside of said container-loading plane (P, P', P") in said operating position due to the effect of said contact.

2. Device as in claim 1, **characterized in that** it comprises automatic thrust means (15a) and automatic return means (23) of said auxiliary guide extension (13, 113, 213), mobile in said direction of feed (X) in order to move said auxiliary guide extension (13, 113, 213) between said retracted position and said operating position, and vice versa.

3. Device as in claim 2, **characterized in that** said automatic thrust means comprise said thrust element (15a) and said return means comprise a return element (23), solidly mobile with said translator slider (15, 115) in said direction of feed (X), **in that** said auxiliary guide extension (13, 113, 213) comprises at least one abutment element (13c), **and in that** said return element (23) is interfering with the bulk of said abutment element (13c) and configured to contact said abutment element (13c) in order to retract said auxiliary guide extension (13, 113, 213) inside said container-loading plane (P, P', P") in said retracted position.

4. Device as in claim 1, 2 or 3, **characterized in that** said translator slider (15, 115) comprises a gripping member (22) provided with a cam profile (22d) sliding on a base (11a) of said containers (11) and a gripping tooth (22c) protruding from said gripping member (22) and mobile with respect to said base (11a), said cam profile (22d) being configured to move said gripping tooth (22c) from a position of non-interference with said base (11a) to a position of interference therewith.

5. Device as in any of the claims from 1 to 4, **characterized in that** it comprises a drive motor (16), and at least one motion transfer device (17), connected to the drive motor (16) and to the translator slider (15), said transfer device (17) being driven by the drive motor (16) and transmitting to the translator slider (15) the motion imparted by the drive motor (16).

6. Device as in claim 5, **characterized in that** said at least one transfer device (17) is the closed ring type and defines a closed trajectory with an upper segment (17a) rectilinear and parallel both to the container-loading plane (P) and to the direction of feed (X) and a lower trajectory segment (17b), which, connected to the upper segment (17a), completes the closed-ring development of the transfer device (17), wherein the upper segment (17a) acts as a rectilinear guide along which the translator slider (15) slides in a guided manner in the direction of feed (X).

7. Device as in claims 3 and 6, **characterized in that** said return element (23) is attached to the at least one transfer device (17), and solidly mobile with it along said closed trajectory.

8. Device as in claim 7, **characterized in that** the return element (23) protrudes from the transfer device (17) in a substantially orthogonal manner to said closed trajectory by a quantity sufficient to contact a support wall (13c) of the frame (13a) when positioned in the upper segment (17a).

9. Device as in claim 8, **characterized in that** said support wall (13c) is located in the lower part of the frame (13a) of the auxiliary guide extension (13) and acts as an abutment element between the return element (23) and the auxiliary guide extension (13), the contact between the return element (23) and the support wall (13c) allowing to automatically move the auxiliary guide extension (13) from its operating position to the retracted position, by moving the translator slider (15) away from the machine (100, 100a, 100b).

10. Device as in any of the claims from 1 to 9, **characterized in that** said thrust element is a thrust surface (15a) of the translator slider (15), with a shape and position, below the container-loading plane (P), coordinated with the shape and position of said abutment element (13b).

11. Device as in any of the claims from 1 to 10, **characterized in that** said abutment element is an abutment surface (13b) with which the frame (13a) of the auxiliary guide extension (13) is provided at the rear.

12. Device as in any of the claims from 1 to 11, **characterized in that** the translator slider (15) comprises a drawing arm (21) provided with a gripping member (22) at its terminal end.

13. Device as in claim 12, **characterized in that** the gripping member (22) is pivoted to the drawing arm (21) and is provided with a first portion (22a) and a second portion (22b) positioned on opposite sides of the pivoting point in the direction of feed (X).

14. Device as in claim 13, **characterized in that** the first portion (22a) and the second portion (22b) are configured to define an asymmetrical balanced condition of the two portions (22a, 22b) with respect to the pivoting.

15. Device as in claim 14, **characterized in that** the gripping member (22) is configured to have a balanced position in which the first portion (22a) is lowered and a normally raised condition of the second portion (22b) with respect to the first portion (22a).

16. Device as in claim 13, 14 or 15, **characterized in that** the second portion (22b) comprises a gripping tooth (22c), protruding from the upper part of the second portion (22b) and transverse with respect to the container-loading plane (P) and to the direction of feed (X).

17. Device as in any of the claims from 13 to 16, **characterized in that** the second portion (22b) comprises a cam profile (22d) configured to slide on a base (11a) of the container (11) during the motion of the translator slider (15) toward the machine (100, 100a, 100b), wherein the sliding of the cam profile (22d) on the base (11a) of the container (11) determines the rotation of the gripping member (22) with respect to its pivoting point, and a lowering of the second portion (22b) with corresponding lifting of the first portion (22a).

18. Device as in any of the claims from 1 to 17, **characterized in that** said motorized linear guide rollers (12, 112, 212) have drawing elements that include silicone rings (12a), configured to contact a base (11a) of the containers (11) and to draw them by friction.

19. Device as in any of the claims from 1 to 18, **characterized in that** said motorized linear guide rollers (12, 112, 212) have a shape that provides lateral shoulders (12b) configured to guide the containers (11) and prevent them from being positioned transverse to the direction of feed (X).

20. Machine for sterilizing objects contained in containers (11), comprising:
- a treatment chamber (101) provided with at least one container-passage aperture (102, 103) in a direction of feed (X),
- a closing door (104, 105) mobile on a lying plane transverse to said direction of feed (X) to close said container-passage aperture (102, 103),
- a device (10, 110, 210) as in any of the claims from 1 to 19.

21. Apparatus for sterilizing objects contained in containers (11) comprising a battery of machines (100, 100a, 100b) for sterilizing said objects, according to claim 20, said machines (100, 100a, 100b) being adjacent and parallel in a work direction (Y).

22. Apparatus as in claim 21, wherein said direction of feed (X) is transverse with respect to said work direction (Y).

23. Apparatus as in claim 21 or 22, wherein said device (10, 110, 210) is mobile frontally to said sterilization machines (100, 100a, 100b) in said work direction (Y), so as to selectively position itself in proximity to one or another of the container-passage apertures (102, 103) in order to introduce and extract said containers (11) with respect to the corresponding treatment chamber (101) in said direction of feed (X).

24. Apparatus as in claim 21, 22 or 23, wherein said machines comprise first machines (100a) and second machines (100b) having a smaller extension than said first machines (100a) in said direction of feed (X), each of said first (100a) and second (100b) machines having two container-passage apertures (102, 103) opposite each other in said direction of feed (X), said second machines (100b) being aligned to said first machines (100a) with respect to one of said container-passage apertures (102, 103), wherein said apparatus comprises a first device (110) sliding in said work direction (Y) and serving said container-passage apertures (102) that are aligned with each other, a second device (10) sliding in said work direction (Y) on an opposite side of said first machines (100a) and second machines (100b) with respect to said first device (110) and serving the container-passage apertures (103) opposite said aligned container-passage apertures (102), and a third device (210) interposed between said second device (10) and the container-passage aperture (103) of each of said second machines (100b) not aligned with the corresponding container-passage apertures (103) of said first machines (100a).

## Patentansprüche

1. Vorrichtung zur Einführung und Entnahme von Behältern (11) in Bezug auf eine Sterilisationsmaschine (100, 100a, 100b) in einer Zuführrichtung (X), umfassend
- eine Behälter-Ladeebene (P, P', P"), die einen Bewegungsweg definiert, und motorisch angetriebene Linearführungsrollen (12, 112, 212), die von einem Stützrahmen (10a) gestützt, entlang der Behälter-Ladeebene (P, P', P") angeordnet und dafür eingerichtet sind, um die Behälter (11) in einer geführten Weise entlang des Bewegungswegs in der Zuführrichtung (X) zu bewegen,
**dadurch gekennzeichnet, dass** sie auch Folgendes umfasst
- eine Hilfsführungsverlängerung (13, 113, 213), die mit der Behälter-Ladeebene (P, P', P") verbunden ist, und die einen Rahmen (13a) umfasst, der mit dem Stützrahmen (10a) verbunden ist, zumindest ein Anschlagelement (13b) und Hilfsführungsrollen (14), wobei der Rahmen (13a) zwischen einer ersten eingezogenen Stellung, in der der Rahmen (13a) in dem Außenmaß der Behälter-Ladeebene (P, P', P") eingeschlossen ist, entweder ausgerichtet zu oder weiter innen liegend in Bezug auf eine Vorderkante des Stützrahmens (10a) und in der die Hilfsführungsrollen (14) koplanar in Bezug auf die motorisch angetriebenen Linearführungsrollen (12, 112, 212) sind, und einer zweiten Betriebsstellung, in der der Rahmen (13a) zumindest teilweise von der Behälter-Ladeebene (P, P', P") hervorstehend ist, um eine Verlängerung des Bewegungswegs zu definieren, gleitend beweglich ist;
- einen Übertragungsschieber (15, 115), der in der Zuführrichtung (X) beweglich ist, um die Behälter (11) in Bezug auf die Maschine (100, 100a, 100b) selektiv einzuführen und zu entnehmen, und der mit einem Schubelement (15a) versehen ist, das dafür eingerichtet ist, um mit dem Außenmaß des Anschlagelementes (13b) zu interferieren, und das dafür eingerichtet ist, um das Anschlagelement (13b) zu kontaktieren und die Hilfsführungsverlängerung (13, 113, 213) in Richtung auf das Äußere der Behälter-Ladeebene (P, P', P") in der Betriebsstellung wegen der Wirkung des Kontakts automatisch zu schieben.

2. Vorrichtung nach Anspruch 1, **dadurch gekennzeichnet, dass** sie automatische Schiebemittel (15a) und automatische Rückholmittel (23) der Hilfsführungsverlängerung (13, 113, 213) umfasst, die in der Zuführrichtung (X) beweglich sind, um die Hilfsführungsverlängerung (13, 113, 213) zwischen der eingezogenen Stellung und der Betriebsstellung und umgekehrt zu bewegen.

3. Vorrichtung nach Anspruch 2, **dadurch gekennzeichnet, dass** die automatischen Schiebemittel das Schubelement (15a) umfassen und die Rückholmittel ein Rückholelement (23) umfassen, das mit dem Übertragungsschieber (15, 115) in der Zuführrichtung (X) fest beweglich ist, **dass** die Hilfsführungsverlängerung (13, 113, 213) mindestens ein Anschlagelement (13c) umfasst, **und dass** das Rückholelement (23) mit dem Außenmaß des Anschlagelementes (13c) interferiert und dafür eingerichtet ist, um das Anschlagelement (13c) zu kontaktieren, um die Hilfsführungsverlängerung (13, 113, 213) innerhalb der Behälter-Ladeebene (P, P', P") in der eingezogenen Stellung einzuziehen.

4. Vorrichtung nach Anspruch 1, 2 oder 3, **dadurch gekennzeichnet, dass** der Übertragungsschieber (15, 115) ein Greifelement (22), das mit einem Nockenprofil (22d) versehen ist, das auf einer Basis (11a) der Behälter (11) gleitet, und einen Greifzahn (22c), der vom Greifelement (22) herausragt und im Hinblick auf die Basis (11a) beweglich ist, umfasst, wobei das Nockenprofil (22d) dafür eingerichtet ist, um den Greifzahn (22c) von einer Nicht-Interferenzposition mit der Basis (11a) zu einer Interferenzposition mit derselben zu bewegen.

5. Vorrichtung nach einem der Ansprüche 1 bis 4, **dadurch gekennzeichnet, dass** sie einen Antriebsmotor (16) und mindestens eine Bewegungsübertragungsvorrichtung (17), die mit dem Antriebsmotor (16) und mit dem Übertragungsschieber (15) verbunden ist, umfasst, wobei die Übertragungsvorrichtung (17) vom Antriebsmotor (16) angetrieben wird und dem Übertragungsschieber (15) die vom Antriebsmotor (16) vermittelte Bewegung überträgt.

6. Vorrichtung nach Anspruch 5, **dadurch gekennzeichnet, dass** die zumindest eine Übertragungsvorrichtung (17) des Typs mit geschlossenem Ring ist und eine geschlossene Bewegungsbahn mit einem oberen Segment (17a), das geradlinig und sowohl zur Behälter-Ladeebene (P) als auch zur Zuführrichtung (X) parallel ist, und ein unteres Bewegungsbahnsegment (17b) definiert, das, mit dem oberen Segment (17a) verbunden, den geschlossenen Ringverlauf der Übertragungsvorrichtung (17) vervollständigt, worin das obere Segment (17a) als geradlinige Führung wirkt, entlang welcher der Übertragungsschieber (15) in einer geführten Weise in der Zuführrichtung (X) gleitet.

7. Vorrichtung nach den Ansprüchen 3 und 6, **dadurch gekennzeichnet, dass** das Rückholelement (23) an der zumindest einen Übertragungsvorrichtung (17) befestigt und mit ihr entlang der geschlossenen Bewegungsbahn fest beweglich ist.

8. Vorrichtung nach Anspruch 7, **dadurch gekennzeichnet, dass** das Rückholelement (23) von der Übertragungsvorrichtung (17) in einer zur geschlossenen Bewegungsbahn im Wesentlichen orthogonalen Weise um ein Maß vorspringt, das ausreichend ist, um eine Stützwand (13c) des Rahmens (13a) dann zu kontaktieren, wenn es im oberen Segment (17a) positioniert ist.

9. Vorrichtung nach Anspruch 8, **dadurch gekennzeichnet, dass** die Stützwand (13c) im unteren Teil des Rahmens (13a) der Hilfsführungsverlängerung (13) angeordnet ist und als Anschlagelement zwischen dem Rückholelement (23) und der Hilfsführungsverlängerung (13) wirkt, wobei der Kontakt zwischen dem Rückholelement (23) und der Stützwand (13c) ermöglicht, die Hilfsführungsverlängerung (13) von ihrer Betriebsstellung zur eingezogenen Stellung dadurch automatisch zu bewegen, dass der Übertragungsschieber (15) weg von der Maschine (100, 100a, 100b) bewegt wird.

10. Vorrichtung nach einem der Ansprüche 1 bis 9, **dadurch gekennzeichnet, dass** das Schubelement eine Schubfläche (15a) des Übertragungsschiebers (15) ist, und zwar mit einer Form und einer Stellung, unterhalb der Behälter-Ladeebene (P), die auf die Form und die Stellung des Anschlagelementes (13b) abgestimmt sind.

11. Vorrichtung nach einem der Ansprüche 1 bis 10, **dadurch gekennzeichnet, dass** das Anschlagelement eine Anschlagfläche (13b) ist, mit der der Rahmen (13a) der Hilfsführungsverlängerung (13) hinten versehen ist.

12. Vorrichtung nach einem der Ansprüche 1 bis 11, **dadurch gekennzeichnet, dass** der Übertragungsschieber (15) einen Zieharm (21) umfasst, der mit einem Greifelement (22) an seinem Abschlussende versehen ist.

13. Vorrichtung nach Anspruch 12, **dadurch gekennzeichnet, dass** das Greifelement (22) am Zieharm (21) schwenkbar angelenkt ist und mit einem ersten Abschnitt (22a) und einem zweiten Abschnitt (22b) versehen ist, die an entgegengesetzten Seiten des Schwenkpunktes in der Zuführrichtung (X) positioniert sind.

14. Vorrichtung nach Anspruch 13, **dadurch gekennzeichnet, dass** der erste Abschnitt (22a) und der zweite Abschnitt (22b) dafür eingerichtet sind, um einen asymmetrischen ausgeglichenen Zustand der beiden Abschnitte (22a, 22b) im Hinblick auf das Schwenken zu definieren.

15. Vorrichtung nach Anspruch 14, **dadurch gekennzeichnet, dass** das Greifelement (22) dafür eingerichtet ist, um eine ausgeglichene Stellung, in der der erste Abschnitt (22a) abgesenkt ist, und einen normalerweise angehobenen Zustand des zweiten Abschnitts (22b) im Hinblick auf den ersten Abschnitt (22a) zu haben.

16. Vorrichtung nach Anspruch 13, 14 oder 15, **dadurch gekennzeichnet, dass** der zweite Abschnitt (22b) einen Greifzahn (22c) umfasst, der vom oberen Teil des zweiten Abschnitts (22b) vorsteht und im Hinblick auf die Behälter-Ladeebene (P) und zur Zuführrichtung (X) quer gerichtet ist.

17. Vorrichtung nach einem der Ansprüche 13 bis 16, **dadurch gekennzeichnet, dass** der zweite Abschnitt (22b) ein Nockenprofil (22d) umfasst, das dafür eingerichtet ist, um auf einer Basis (11a) des Behälters (11) während der Bewegung des Übertragungsschiebers (15) in Richtung auf die Maschine (100, 100a, 100b) zu gleiten, worin das Gleiten des Nockenprofils (22d) auf der Basis (11a) des Behälters (11) die Drehung des Greifelementes (22) im Hinblick auf seinen Schwenkpunkt und eine Senkung des zweiten Abschnitts (22b) mit entsprechender Hebung des ersten Abschnitts (22a) bewirkt.

18. Vorrichtung nach einem der Ansprüche 1 bis 17, **dadurch gekennzeichnet, dass** die motorisch angetriebenen Linearführungsrollen (12, 112, 212) Ziehelemente haben, die Silikonringe (12a) umfassen, die dafür eingerichtet sind, um eine Basis (11a) der Behälter (11) zu kontaktieren und sie durch Reibung zu ziehen.

19. Vorrichtung nach einem der Ansprüche 1 bis 18, **dadurch gekennzeichnet, dass** die motorisch angetriebenen Linearführungsrollen (12, 112, 212) eine Gestalt aufweisen, die seitliche Schultern (12b) vorsieht, die dafür eingerichtet sind, um die Behälter (11) zu führen und zu verhindern, dass diese quer zur Zuführrichtung (X) positioniert werden.

20. Maschine zur Sterilisation von Gegenständen, die in Behältern (11) enthalten sind, umfassend:
- eine Behandlungskammer (101), die mit zumindest einer Behälterdurchgangsöffnung (102, 103) in einer Zuführrichtung (X) versehen ist,
- eine Schließtür (104, 105), die in einer Liegeebene quer zur Zuführrichtung (X) beweglich ist, um die Behälterdurchgangsöffnung (102, 103) zu schließen,
- eine Vorrichtung (10, 110, 210) nach einem der Ansprüche 1 bis 19.

21. Apparat zur Sterilisation von Gegenständen, die in Behältern (11) enthalten sind, umfassend eine Serie von Maschinen (100, 100a, 100b) zur Sterilisation der Gegenstände, nach Anspruch 20, wobei die Maschinen (100, 100a, 100b) in einer Arbeitsrichtung (Y) einander angrenzend und zueinander parallel sind.

22. Apparat nach Anspruch 21, worin die Zuführrichtung (X) zur Arbeitsrichtung (Y) quer gerichtet ist.

23. Apparat nach Anspruch 21 oder 22, worin die Vorrichtung (10, 110, 210) frontal zu den Sterilisationsmaschinen (100, 100a, 100b) in der Arbeitsrichtung (Y) bewegbar ist, so dass sie sich selektiv in der Nähe von der einen oder der anderen der Behälterdurchgangsöffnungen (102, 103) positioniert, um die Behälter (11) im Bezug auf die entsprechende Behandlungskammer (101) in der Zuführrichtung (X) einzuführen und zu entnehmen.

24. Apparat nach Anspruch 21, 22 oder 23, worin die Maschinen erste Maschinen (100a) und zweite Maschinen (100b) umfassen, die einen kleineren Umfang als die ersten Maschinen (100a) in der Zuführrichtung (X) haben, wobei jede der ersten (100a) und der zweiten Maschinen (100b) zwei Behälterdurchgangsöffnungen (102, 103) haben, die in der Zuführrichtung (X) einander gegenüberliegen, wobei die zweiten Maschinen (100b) zu den ersten Maschinen (100a) im Hinblick auf eine der Behälterdurchgangsöffnungen (102, 103) ausgerichtet sind, worin der Apparat eine erste Vorrichtung (110), die in der Arbeitsrichtung (Y) gleitet und die Behälterdurchgangsöffnungen (102) bedient, die zueinander ausgerichtet sind, eine zweite Vorrichtung (10), die in der Arbeitsrichtung (Y) auf einer gegenüberliegenden Seite der ersten Maschinen (100a) und der zweiten Maschinen (100b) im Hinblick auf die erste Vorrichtung (110) gleitet und die Behälterdurchgangsöffnungen (103) bedient, die den zueinander ausgerichteten Behälterdurchgangsöffnungen (102) gegenüberliegen, und eine dritte Vorrichtung (210) umfasst, die zwischen der zweiten Vorrichtung (10) und der Behälterdurchgangsöffnung (103) von jeder der zweiten Maschinen (100b), die zu den entsprechenden Behälterdurchgangsöffnungen (103) der ersten Maschinen (100a) nicht ausgerichtet ist, zwischengeschaltet ist.

## Revendications

1. Dispositif pour introduire et extraire des récipients (11) par rapport à une machine de stérilisation (100, 100a, 100b) dans une direction d'alimentation (X), comprenant :
- un plan de chargement de récipients (P, P', P") définissant un trajet de déplacement, et des galets de guidage linéaires motorisés (12, 112, 212), supportés par un châssis de support (10a), disposés le long du plan de chargement de récipients (P, P', P") et configurés pour déplacer lesdits récipients (11) de manière guidée le long dudit trajet de déplacement dans la direction d'alimentation (X),
**caractérisé en ce qu'**il comprend également
- une extension de guidage auxiliaire (13, 113, 213) reliée audit plan de chargement de récipients (P, P', P") et comprenant un châssis (13a) relié au châssis de support (10a), au moins un élément formant butée (13b) et des galets de guidage auxiliaires (14), ledit châssis (13a) étant mobile de manière coulissante entre une première position rétractée, dans laquelle ledit châssis (13a) est compris dans l'enveloppe du plan de chargement de récipients (P, P', P"), aligné ou plus à l'intérieur par rapport à un bord avant du châssis de support (10a) et dans laquelle les galets de guidage auxiliaires (14) sont coplanaires par rapport aux galets de guidage linéaires motorisés (12, 112, 212), et une deuxième position de fonctionnement, dans laquelle ledit châssis (13a) fait au moins partiellement saillie du plan de chargement de récipients (P, P', P") afin de définir une extension dudit trajet de déplacement ;
- un coulisseau translateur (15, 115) mobile dans ladite direction d'alimentation (X) pour sélectivement extraire ou insérer lesdits récipients (11) par rapport à ladite machine (100, 100a, 100b), et muni d'un élément de poussée (15a) configuré pour interférer avec l'enveloppe dudit élément formant butée (13b) et configuré pour venir en contact avec ledit élément formant butée (13b) et pousser automatiquement ladite extension de guidage auxiliaire (13, 113, 213) vers l'extérieur dudit plan de chargement de récipients (P, P', P") dans ladite position de fonctionnement sous l'effet de ladite venue en contact.

2. Dispositif selon la revendication 1, **caractérisé en ce qu'**il comprend des moyens de poussée automatique (15a) et des moyens de rappel automatique (23) de ladite extension de guidage auxiliaire (13, 113, 213), mobiles dans ladite direction d'alimentation (X) afin de déplacer ladite extension de guidage auxiliaire (13, 113, 213) entre ladite position rétractée et ladite position de fonctionnement, et vice versa.

3. Dispositif selon la revendication 2, **caractérisé en ce que** lesdits moyens de poussée automatique comprennent ledit élément de poussée (15a), et lesdits moyens de rappel comprennent un élément de rappel (23), solidaire dudit coulisseau translateur (15, 115) dans ladite direction d'alimentation (X) et mobile avec celui-ci, **en ce que** ladite extension de guidage auxiliaire (13, 113, 213) comprend au moins un élément formant butée (13c), et **en ce que** ledit élément de rappel (23) interfère avec l'enveloppe dudit élément formant butée (13c) et est configurer pour contacter ledit élément formant butée (13c) afin de rétracter ladite extension de guidage auxiliaire (13, 113, 213) à l'intérieur dudit plan de chargement de récipients (P, P', P") dans ladite position rétractée.

4. Dispositif selon la revendication 1, 2 ou 3, **caractérisé en ce que** ledit coulisseau translateur (15, 115) comprend un organe de préhension (22) pourvu d'un profil de came (22d) coulissant sur une base (11a) desdits récipients (11) et une dent de préhension (22c) faisant saillie dudit élément de préhension (22) et mobile par rapport à ladite base (11a), ledit profil de came (22d) étant configuré pour déplacer ladite dent de préhension (22c) d'une position de non -interférence avec ladite base (11a) jusqu'à une position d'interférence avec celle-ci.

5. Dispositif selon l'une quelconque des revendications 1 à 4, **caractérisé en ce qu'**il comprend un moteur d'entraînement (16), et au moins un dispositif de transfert de mouvement (17), relié au moteur d'entraînement (16) et au coulisseau translateur (15), ledit dispositif de transfert (17) étant entraîné par le moteur d'entraînement (16) et transmettant au coulisseau translateur (15) le mouvement transmis par le moteur d'entraînement (16).

6. Dispositif selon la revendication 5, **caractérisé en ce que** ledit au moins un dispositif de transfert (17) est du type à anneau fermé et définit une trajectoire fermée avec un segment supérieur (17a) rectiligne et parallèle à la fois au plan de chargement de récipients (P) et à la direction d'alimentation (X), et avec un segment de trajectoire inférieur (17b) qui, relié au segment supérieur (17a), achève le développement en anneau fermé du dispositif de transfert (17), le segment supérieur (17a) agissant comme un guide rectiligne le long duquel le coulisseau translateur (15) coulisse de manière guidée dans la direction d'alimentation (X).

7. Dispositif selon les revendications 3 et 6, **caractérisé en ce que** ledit élément de rappel (23) est fixé audit au moins un dispositif de transfert (17) et est mobile solidairement à lui le long de ladite trajectoire fermée.

8. Dispositif selon la revendication 7, **caractérisé en ce que** l'élément de rappel (23) fait saillie du dispositif de transfert (17) d'une manière sensiblement orthogonale à ladite trajectoire fermée, sur une distance suffisante pour venir en contact avec une paroi de support (13c) du châssis (13a) lorsqu'il est positionné dans le segment supérieur (17a).

9. Dispositif selon la revendication 8, **caractérisé en ce que** ladite paroi de support (13c) est située dans la partie inférieure du châssis (13a) de l'extension de guidage auxiliaire (13) et agit en tant qu'élément de butée entre l'élément de rappel (23) et l'extension de guidage auxiliaire (13), le contact entre l'élément de rappel (23) et la paroi de support (13c) permettant de déplacer automatiquement l'extension de guidage auxiliaire (13) de sa position de fonctionnement à la position rétractée (15) en déplaçant le coulisseau translateur (15) de façon à l'éloigner de la machine (100, 100a, 100b).

10. Dispositif selon l'une quelconque des revendications 1 à 9, **caractérisé en ce que** ledit élément de poussée est une surface de poussée (15a) du coulisseau translateur (15), ayant une forme et une position, en dessous du plan de chargement de récipients (P), coordonnée avec la forme et la position dudit élément formant butée (13b).

11. Dispositif selon l'une quelconque des revendications 1 à 10, **caractérisé en ce que** ledit élément formant butée est une surface de butée (13b) dont le châssis (13a) de l'extension de guidage auxiliaire (13) est pourvu à l'arrière de lui.

12. Dispositif selon l'une quelconque des revendications 1 à 11, **caractérisé en ce que** le coulisseau translateur (15) comprend un bras de retrait (21) pourvu d'un organe de préhension (22) à son extrémité terminale.

13. Dispositif selon la revendication 12, **caractérisé en ce que** l'organe de préhension (22) pivote sur le bras de retrait (21) et comporte une première partie (22a) et une deuxième partie (22b) disposées sur des côtés opposés du point de pivotement dans la direction d'alimentation (X).

14. Dispositif selon la revendication 13, **caractérisé en ce que** la première partie (22a) et la deuxième partie (22b) sont configurées pour définir une condition asymétrique équilibrée des deux parties (22a, 22b) par rapport au pivotement.

15. Dispositif selon la revendication 14, **caractérisé en ce que** l'organe de préhension (22) est configuré pour avoir une position équilibrée dans laquelle la première partie (22a) est abaissée et une condition normalement relevée de la deuxième partie (22b) par rapport à la première partie (22a).

16. Dispositif selon la revendication 13, 14 ou 15, **caractérisé en ce que** la deuxième partie (22b) comprend une dent de préhension (22c), faisant saillie de la partie supérieure de la deuxième partie (22b) et transversale par rapport au plan (P) de chargement de récipients et à la direction d'alimentation (X).

17. Dispositif selon l'une quelconque des revendications 13 à 16, **caractérisé en ce que** la deuxième partie (22b) comprend un profil de came (22d) configuré pour coulisser sur une base (11a) du récipient (11) pendant le mouvement du coulisseau translateur (15) vers la machine (100, 100a, 100b), le coulissement du profil de came (22d) sur la base (11a) du conteneur (11) déterminant la rotation de l'organe de préhension (22) avec par rapport à son point de pivotement, et un abaissement de la deuxième partie (22b) avec soulèvement correspondant de la première partie (22a).

18. Dispositif selon l'une quelconque des revendications 1 à 17, **caractérisé en ce que** lesdits galets de guidage linéaires motorisés (12, 112, 212) comportent des éléments de tirage comportant des anneaux en silicone (12a), configurés pour venir en contact avec une base (11a) des récipients (11) et pour les tirer par friction.

19. Dispositif selon l'une quelconque des revendications 1 à 18, **caractérisé en ce que** lesdits galets de guidage linéaires motorisés (12, 112, 212) ont une forme qui présente des épaulements latéraux (12b) configurés pour guider les récipients (11) et pour empêcher ces derniers d'être positionnés transversalement à la direction d'alimentation (X).

20. Machine de stérilisation d'objets contenus dans des récipients (11), comprenant :
- une chambre de traitement (101) pourvue d'au moins une ouverture (102, 103) de passage de récipient dans une direction d'alimentation (X),
- une porte de fermeture (104, 105) mobile sur un plan d'extension transversal à ladite direction d'alimentation (X) pour fermer ladite ouverture (102, 103) de passage de récipient,
- un dispositif (10, 110, 210) selon l'une quelconque des revendications 1 à 19.

21. Appareil de stérilisation d'objets contenus dans des récipients (11) comprenant une batterie de machines (100, 100a, 100b) pour stériliser lesdits objets, selon la revendication 20, lesdites machines (100, 100a, 100b) étant adjacentes et parallèles dans une direction de travail (Y).

22. Appareil selon la revendication 21, dans lequel ladite direction d'alimentation (X) est transversale par rapport à ladite direction de travail (Y).

23. Appareil selon la revendication 21 ou la revendication 22, dans lequel ledit dispositif (10, 110, 210) est mobile frontalement par rapport auxdites machines de stérilisation (100, 100a, 100b) dans ladite direction de travail (Y), de façon à se positionner sélectivement à proximité de l'un ou l'autre des ouvertures (102, 103) de passage de récipients afin d'introduire et d'extraire lesdits récipients (11) par rapport à la chambre (101) de traitement correspondante dans ladite direction d'alimentation (X).

24. Appareil selon la revendication 21, 22 ou 23, dans lequel lesdites machines comprennent des premières machines (100a) et des deuxièmes machines (100b) ayant une extension plus petite que lesdites premières machines (100a) dans ladite direction d'alimentation (X), chacune desdites premières machines (100a) et deuxièmes (100b) machines ayant deux ouvertures (102, 103) de passage de récipients opposées l'une à l'autre dans ladite direction d'alimentation (X), lesdites deuxièmes machines (100b) étant alignées avec lesdites premières machines (100a) par rapport à l'une desdites ouvertures (102, 103) de passage de récipients, ledit appareil comprenant un premier dispositif (110) coulissant dans ladite direction de travail (Y) et desservant lesdites ouvertures (102) de passage de récipients qui sont alignées les unes aux autres, un deuxième dispositif (10) coulissant dans ladite direction de travail (Y) sur un côté opposé desdites premières machines (100a) et deuxièmes machines (100b) par rapport audit premier dispositif (110) et desservant les ouvertures (103) de passage de récipients opposées auxdites ouvertures (102) alignées de passage de récipients, et un troisième dispositif (210) interposé entre ledit deuxième dispositif (10) et l'ouverture (103) de passage de récipients de chacune desdites deuxièmes machines (100b) non alignées avec les ouvertures (103) correspondantes de passage de récipients desdites premières machines (100a).
